# EUROPEAN PATENT APPLICATION

(11) **EP 2 339 031 A1**
(43) Date of publication of application: **29.06.2011**
(21) Application number: 10014685.1
(22) Date of filing: 17.11.2010
(51) Int. Cl.: C12Q 1/68, A01K 67/027, C12N 15/85

(54) **Transgenic animal for screening of compounds that modulate cell proliferation, and its use in the pharmaceutical field**

(30) Priority: 17.11.2009 IT MI20092023
(71) Applicant: TOP (Transgenic Operative Products) S.R.L., 26900 Lodi (LO) (IT); Istituti Fisioterapici Ospitalieri, 00144 Roma (IT)
(72) Inventor: Maggi, Adriana, 20123 Milano (IT); Ciana, Paolo, 28883 Gravellona Toce (IT); Piaggio, Giulia, 00196 Roma (IT); Goeman, Frauke, 00124 Roma (IT); Sacchi, Ada, 00100 Roma (IT); Tiveron, Cecilia, 00100 Roma (IT)
(74) Representative: Appoloni, Romano

(57) **Abstract**

Summary

The present invention relates to a transgenic non-human mammalian animal whose genome incorporates a biosensor of the activity of modulators of cell proliferation consisting of a DNA sequence that comprises the luciferase reporter transgene ligated downstream of a promoter sequence of the CYCB2 gene, this sequence promoter/reporter being flanked by HS4 insulator sequences at each of its 3'and 5' sides.

The transgenic animal is proposed for the screening of compounds with pharmacologically relevant activities.

The invention provides for a further use of the animal line for the study and toxicological evaluation of compounds with tumorigenic activity, and also for the study and evaluation of compounds with chemopreventive anti-cancer activity.

SEQUENCE LISTING

<110> TOP (TRANSGENIC OPERATIVE PRODUCTS) S.r.l.
ISTITUTI FISIOTERAPICI OSPITALIERI

<120> TRANSGENIC ANIMAL FOR SCREENING OF COMPOUNDS THAT MODULATE CELL
PROLIFERATION, AND ITS USE IN THE PHARMACEUTICAL FIELD

<130> 9491PTEP

<150> MI2009A002023
<151> 2009-11-17

<160> 6

<170> PatentIn version 3.5

<210> 1
<211> 312
<212> DNA
<213> Mus musculus

<220>
<221> promoter
<222> (1)..(312)
<223> Cyclin B2; GenBank n° AY88998

<400> 1
tatgtcctta ggtgatggct ccccactcct acccctaaaa ttaagacaat cttttccagt 60

aggaggaaaa attattttat ttaatatcag ggactagaat ttgaaaataa gactgtagac 120

aaggaaacaa caaagcctgg tggcctcgct ggttgctatg acaagcaaat acaagccagc 180

caatcaacgt gcagaaaggc cttccagtct agccaatggg ttgcgcgcgc cctgcgtgcg 240

tctacccaat agtgcgtcag cggcgcggta tttgaatcgc ggaccgggcg gtggacgcgg 300

agcggcgggg cc 312

<210> 2
<211> 1653
<212> DNA
<213> Photinus pyralis

<400> 2
atggaagacg ccaaaaacat aaagaaaggc ccggcgccat tctatcctct agaggatgga 60

accgctggag agcaactgca taaggctatg aagagatacg ccctggttcc tggaacaatt 120

gcttttacag atgcacatat cgaggtgaac atcacgtacg cggaatactt cgaaatgtcc 180

gttcggttgg cagaagctat gaaacgatat gggctgaata caaatcacag aatcgtcgta 240

tgcagtgaaa actctcttca attctttatg ccggtgttgg gcgcgttatt tatcggagtt 300

gcagttgcgc ccgcgaacga catttataat gaacgtgaat tgctcaacag tatgaacatt 360

tcgcagccta ccgtagtgtt tgtttccaaa aaggggttgc aaaaaatttt gaacgtgcaa 420

aaaaaattac caataatcca gaaaattatt atcatggatt ctaaaacgga ttaccaggga 480

tttcagtcga tgtacacgtt cgtcacatct catctacctc ccggttttaa tgaatacgat 540

tttgtaccag agtcctttga tcgtgacaaa acaattgcac tgataatgaa ttcctctgga 600

tctactgggt tacctaaggg tgtggccctt ccgcatagaa ctgcctgcgt cagattctcg 660
catgccagag atcctatttt tggcaatcaa atcattccgg atactgcgat tttaagtgtt 720
gttccattcc atcacggttt tggaatgttt actacactcg gatatttgat atgtggattt 780
cgagtcgtct taatgtatag atttgaagaa gagctgtttt tacgatccct tcaggattac 840
aaaattcaaa gtgcgttgct agtaccaacc ctattttcat tcttcgccaa aagcactctg 900
attgacaaat acgatttatc taatttacac gaaattgctt ctgggggcgc acctctttcg 960
aaagaagtcg gggaagcggt tgcaaaacgc ttccatcttc cagggatacg acaaggatat 1020
gggctcactg agactacatc agctattctg attacacccg agggggatga taaaccgggc 1080
gcggtcggta aagttgttcc attttttgaa gcgaaggttg tggatctgga taccgggaaa 1140
acgctgggcg ttaatcagag aggcgaatta tgtgtcagag gacctatgat tatgtccggt 1200
tatgtaaaca atccggaagc gaccaacgcc ttgattgaca aggatggatg gctacattct 1260
ggagacatag cttactggga cgaagacgaa cacttcttca tagttgaccg cttgaagtct 1320
ttaattaaat acaaaggata tcaggtggcc cccgctgaat tggaatcgat attgttacaa 1380
caccccaaca tcttcgacgc gggcgtggca ggtcttcccg acgatgacgc cggtgaactt 1440
cccgccgccg ttgttgtttt ggagcacgga aagacgatga cggaaaaaga gatcgtggat 1500
tacgtcgcca gtcaagtaac aaccgcgaaa aagttgcgcg gaggagttgt gtttgtggac 1560
gaagtaccga aaggtcttac cggaaaactc gacgcaagaa aaatcagaga gatcctcata 1620
aaggccaaga agggcggaaa gtccaaattg taa 1653

<210> 3
<211> 550
<212> PRT
<213> Photinus pyralis

<400> 3

Met Glu Asp Ala Lys Asn Ile Lys Lys Gly Pro Ala Pro Phe Tyr Pro
1               5                   10                  15

Leu Glu Asp Gly Thr Ala Gly Glu Gln Leu His Lys Ala Met Lys Arg
            20                  25                  30

Tyr Ala Leu Val Pro Gly Thr Ile Ala Phe Thr Asp Ala His Ile Glu
        35                  40                  45

Val Asn Ile Thr Tyr Ala Glu Tyr Phe Glu Met Ser Val Arg Leu Ala
    50                  55                  60

Glu Ala Met Lys Arg Tyr Gly Leu Asn Thr Asn His Arg Ile Val Val
65                  70                  75                  80

Cys Ser Glu Asn Ser Leu Gln Phe Phe Met Pro Val Leu Gly Ala Leu
                85                  90                  95

Phe Ile Gly Val Ala Val Ala Pro Ala Asn Asp Ile Tyr Asn Glu Arg
            100                 105                 110

Glu Leu Leu Asn Ser Met Asn Ile Ser Gln Pro Thr Val Val Phe Val
        115                 120                 125

Ser Lys Lys Gly Leu Gln Lys Ile Leu Asn Val Gln Lys Lys Leu Pro
    130                 135                 140

Ile Ile Gln Lys Ile Ile Ile Met Asp Ser Lys Thr Asp Tyr Gln Gly
145                 150                 155                 160

Phe Gln Ser Met Tyr Thr Phe Val Thr Ser His Leu Pro Pro Gly Phe
                165                 170                 175

Asn Glu Tyr Asp Phe Val Pro Glu Ser Phe Asp Arg Asp Lys Thr Ile
            180                 185                 190

Ala Leu Ile Met Asn Ser Ser Gly Ser Thr Gly Leu Pro Lys Gly Val
        195                 200                 205

Ala Leu Pro His Arg Thr Ala Cys Val Arg Phe Ser His Ala Arg Asp
    210                 215                 220

Pro Ile Phe Gly Asn Gln Ile Ile Pro Asp Thr Ala Ile Leu Ser Val
225                 230                 235                 240

Val Pro Phe His His Gly Phe Gly Met Phe Thr Thr Leu Gly Tyr Leu
                245                 250                 255

Ile Cys Gly Phe Arg Val Val Leu Met Tyr Arg Phe Glu Glu Glu Leu
            260                 265                 270

Phe Leu Arg Ser Leu Gln Asp Tyr Lys Ile Gln Ser Ala Leu Leu Val
        275                 280                 285

Pro Thr Leu Phe Ser Phe Phe Ala Lys Ser Thr Leu Ile Asp Lys Tyr
    290                 295                 300

Asp Leu Ser Asn Leu His Glu Ile Ala Ser Gly Gly Ala Pro Leu Ser
305                 310                 315                 320

Lys Glu Val Gly Glu Ala Val Ala Lys Arg Phe His Leu Pro Gly Ile
                325                 330                 335

Arg Gln Gly Tyr Gly Leu Thr Glu Thr Thr Ser Ala Ile Leu Ile Thr
            340                 345                 350

Pro Glu Gly Asp Asp Lys Pro Gly Ala Val Gly Lys Val Val Pro Phe
        355                 360                 365

Phe Glu Ala Lys Val Val Asp Leu Asp Thr Gly Lys Thr Leu Gly Val
    370                 375                 380

Asn Gln Arg Gly Glu Leu Cys Val Arg Gly Pro Met Ile Met Ser Gly
385                 390                 395                 400

Tyr Val Asn Asn Pro Glu Ala Thr Asn Ala Leu Ile Asp Lys Asp Gly
                405                 410                 415

Trp Leu His Ser Gly Asp Ile Ala Tyr Trp Asp Glu Asp Glu His Phe
            420                 425                 430

Phe Ile Val Asp Arg Leu Lys Ser Leu Ile Lys Tyr Lys Gly Tyr Gln
        435                 440                 445

Val Ala Pro Ala Glu Leu Glu Ser Ile Leu Leu Gln His Pro Asn Ile
    450                 455                 460

Phe Asp Ala Gly Val Ala Gly Leu Pro Asp Asp Asp Ala Gly Glu Leu
465                 470                 475                 480

Pro Ala Ala Val Val Val Leu Glu His Gly Lys Thr Met Thr Glu Lys
                485                 490                 495

Glu Ile Val Asp Tyr Val Ala Ser Gln Val Thr Thr Ala Lys Lys Leu
            500                 505                 510

Arg Gly Gly Val Val Phe Val Asp Glu Val Pro Lys Gly Leu Thr Gly
        515                 520                 525

Lys Leu Asp Ala Arg Lys Ile Arg Glu Ile Leu Ile Lys Ala Lys Lys
    530                 535                 540

Gly Gly Lys Ser Lys Leu
545                 550

<210>  4
<211>  2392
<212>  DNA
<213>  Gallus gallus

<220>
<221>  misc_feature
<222>  (1)..(2392)
<223>  Insulator sequence from b-globin-gene: GenBank N° AJ277959

<400>  4
agggacagcc cccccccaaa gcccccaggg atgtaattac gtccctcccc cgctaggggg      60

cagcagcgag ccgcccgggg ctccgctccg gtccggcgct ccccccgcat ccccgagccg     120

gcagcgtgcg gggacagccc gggcacgggg aaggtggcac gggatcgctt tcctctgaac     180

gcttctcgct gctctttgag cctgcagaca cctgggggat acggggaaaa agctttaggc     240

tgaaagagag atttagaatg acagaatcat agaacgcctg ggttgcaaag gagcacagtg 300
ctcatccaga tccaaccccc tgctatgtgc aggtcatcaa ccagcagccc agcgcgtcag 360
agccacatcc agcctggcct tgaatgcctg cctgcaggga tggggcatcc acagcctcct 420
tgggcaacct gttcagtgcg tcaccaccct ctggggaaaa actgcctcct catatccaac 480
ccaaacctcc cctgtctcag tgtaaagcca ttcccccttg tcctatcaag ggggagtttg 540
ctgtgacatt gttggtctgg ggtgacacat gtttgccaat tcagtgctca cggagaggca 600
gatcttggga taaggaagtg caggacagca tggacgtgga catgcaggtg ttgaggctct 660
ggacactcca agtcacagcg ttcagaacag ccttaaggtc aagaagatag gatagaagga 720
caaagagcaa gttaaaaccc agcatggaga ggagcacaaa aaggccacag acactgctgg 780
tccctgtgtc tgagcctgca tgtttgatgg tgtctggatg caagcagaag gggtggaaga 840
gcttgcctgg agagatacag gctgggtcgt aggactggga caggcagctg gagaattgcc 900
atgtagatgt tcatacaatc gtcaaatcat gaaggctgga aaagctccaa gatccccaag 960
accaacccca acccacccac cgtgccactg gccatgtccc tcagtgccac atccccacag 1020
ttcttcatca cctccaggga cggtgacccc ctcctccgtg gcagctgtgc cactgcagca 1080
ccgctctttg gagaaggtaa atcttgctaa atccagcccg accctcccct ggcacaacgt 1140
aaggccatta tctctcatcc aactccagga cggagtcagt gagaatattt ctagagagct 1200
cacggggaca gccccccccc aaagccccca gggatgtaat tacgtccctc ccccgctagg 1260
gggcagcagc gagccgcccg gggctccgct ccggtccggc gctccccccg catccccgag 1320
ccggcagcgt gcggggacag cccgggcacg gggaaggtgg cacgggatcg ctttcctctg 1380
aacgcttctc gctgctcttt gagcctgcag acacctgggg gatacgggga aaaagcttta 1440
ggctgaaaga gagatttaga atgacagaat catagaacgc ctgggttgca aaggagcaca 1500
gtgctcatcc agatccaacc ccctgctatg tgcaggtcat caaccagcag cccagcgcgt 1560
cagagccaca tccagcctgg ccttgaatgc ctgcctgcag ggatggggca tccacagcct 1620
ccttgggcaa cctgttcagt gcgtcaccac cctctgggga aaaactgcct cctcatatcc 1680
aacccaaacc tcccctgtct cagtgtaaag ccattccccc ttgtcctatc aagggggagt 1740
ttgctgtgac attgttggtc tggggtgaca catgtttgcc aattcagtgc tcacggagag 1800
gcagatcttg ggataaggaa gtgcaggaca gcatggacgt ggacatgcag gtgttgaggc 1860
tctggacact ccaagtcaca gcgttcagaa cagccttaag gtcaagaaga taggatagaa 1920
ggacaaagag caagttaaaa cccagcatgg agaggagcac aaaaaggcca cagacactgc 1980
tggtccctgt gtctgagcct gcatgtttga tggtgtctgg atgcaagcag aaggggtgga 2040
agagcttgcc tggagagata caggctgggt cgtaggactg ggacaggcag ctggagaatt 2100
gccatgtaga tgttcataca atcgtcaaat catgaaggct ggaaaagctc caagatcccc 2160
aagaccaacc ccaacccacc caccgtgcca ctggccatgt ccctcagtgc cacatcccca 2220
cagttcttca tcacctccag ggacggtgac cccctcctcc gtggcagctg tgccactgca 2280

gcaccgctct ttggagaagg taaatcttgc taaatccagc ccgaccctcc cctggcacaa     2340

cgtaaggcca ttatctctca tccaactcca ggacggagtc agtgagaata tt     2392

<210>  5
<211>  32
<212>  DNA
<213>  Mus musculus

<400>  5
tgtagacaag gaaacaacaa agcctggtgg cc     32

<210>  6
<211>  29
<212>  DNA
<213>  Artificial Sequence

<220>
<223>  PCR Primer

<400>  6
   ggcgtcttcc attttaccaa cagtaccgg     29

## Description

The present invention relates to a transgenic animal for the screening of compounds endowed with activities of pharmacological interest, and to its use in the pharmaceutical field.

A dysregulation of signal transduction pathways involved In cell cycle progression through different phases, resulting in hyper-or hypo-proliferation, is at the basis of several human diseases. For this reason, active compounds on cell proliferation are the subject of great pharmacological Interest.

Currently there are no known systems that make possible to measure in a simple, direct and ubiquitous way, and in all tissues of an animal model, the activity of compounds able to modulate cell proliferation. Instead it would be desirable to obtain the activity profile of the study compounds both in living animals, especially by application of *in vivo* non-invasive imaging techniques, and in any kind of explanted tissue by *ex vivo* imaging techniques or biochemical determination of enzymatic activities.

Currently most systems used for pre-clinical screening of compounds with anti- or pro-proliferative activity are based both on cell culture models and on transplants in immunodeficient animals.

Instead the evaluation of proliferative activity in normal or tumor tissues in an animal model provides for the histochemical determination of endogenously expressed proliferation markers, such as the nuclear protein Ki67, or the administration of markers, such as bromodeoxyuridine. In this case the analysis, in addition to being laborious and involving the sacrifice of the animal, does not allow an easy quantification of proliferative activity. A method of analysis by *in vivo* imaging or by biochemical analysis of a reporter activity would be a good alternative because it is rapid, inexpensive, applicable to living animals, thus allowing in all cases a quantitative measurement of the pharmacological activity under study.

U.S. 7041869 proposed a transgenic mouse model that can be used to monitor, by *in vivo* imaging, the areas of tumor growth where bioluminescence is produced by the luciferase (Luc) reporter gene driven by E2F-1 promoter, acting as cell cycle biosensor. Use of this transgenic mouse model for drug screening is limited by the fact that, on the one hand, the analytical application is restricted to tumor tissues and, on the other hand, the biosensor constructed in this way cannot measure accurately the proliferative state in all tissues of the animal in an ubiquitous manner. In fact, a biosensor such as the E2F-Luc reporter transgene can only detect proliferation of specific cancer cells carrying an inactive Rb tumor suppressor.

It is instead desired a drug screening method that overcomes these limitations and allows measurement of the proliferative status of all cells, hence of the activity of the compound under investigation on cell proliferation in all tissues, both normal and cancerous.

To achieve this goal, the present invention proposes a mammalian non-human transgenic animal whose genome incorporates, as a biosensor of the activity of cell proliferation modulators, a DNA sequence comprising the luciferase reporter transgene ligated downstream of the sequence of the CYCB2 gene promoter, where the whole promoter/reporter sequence is flanked by HS4 insulator sequences at both 3' and 5' sides.

In a preferred embodiment, the transgenic animal according to the invention is a mouse.

The characterization of the sequences mentioned above is reported below, including the GenBank accession number:
- CYCB2, Mus musculus, GenBank: AY788998.1 - Reference: Lange-zu D. et al., FEBS Lett. 484 (2), 77-81 (2000)
- Luciferase, Photinus pyralis. GenBank: M15077.1 - Reference: de Wet, J.R., Mol. Cell. Biol. 7 (2), 725-737 (1987)
- HS4 insulator, beta-globin gene Gallus gallus, GenBank: AJ277959 - Reference: Ciana P., Mol. Endocrinol. 15 (7), 1104-1113 (2001), corresponding to SEQ ID NO:4.

The invention provides for a first use of a transgenic non-human mammalian animal in order to study and develop compounds of potential pharmacological interest with inhibitory activity on cell proliferation. In this context, the animal is expected to be used especially to investigate and develop compounds with antitumor activity and compounds with anti-inflammatory activity.

The invention involves a further use of the animal to investigate and develop compounds of potential pharmacological interest with cell proliferation inducing activity. In particular, in this context, it is envisaged its use to study and develop compounds of potential pharmacological interest such as growth factors that selectively stimulate stem cells proliferation.

The invention provides for a further use of the transgenic non-human mammalian animal for the study and toxicological evaluation of compounds with tumorigenic activity.

The invention provides for a further use of the transgenic non-human mammalian animal for the study and evaluation of compounds with anti-cancer chemopreventive activity.

The invention relates also to a method for evaluation of the above discussed activities, based on the generation of a transgenic non-human mammalian animal wherein cell proliferation can be simultaneously monitored in all normal or tumor tissues. The method is generally characterized by the following steps:
- Generate a construct consisting of a nucleotide sequence comprising a luciferase reporter transgene ligated downstream of a specific sequence of the CYCB2 gene promoter, in which both 3' and 5' sides of the promoter/reporter sequence are flanked by HS4 insulator sequences;
- Incorporate this construct into the genome of the animal;
- Treat individuals of this transgenic animal line with said modulator of cell proliferation;
- Assess the amount of luciferase produced *in vivo* by imaging assays performed on the animal or *ex vivo* by enzyme activity assays performed on tissues and cells;
- Compare the luminescence emission of said individual animals treated with said modulator of cell proliferation with the other untreated individuals of the same transgenic animal line.

The following experimental part, in reference to the enclosed figures, is described for a better understanding of the characteristics and advantages of the invention and should not be intended in any way to limit the scope of the invention itself.

Figure 1 schematically shows the luciferase reporter transgene construct, comprising the HS4 insulator sequences flanking said luciferase reporter transgene ligated to the CYCB2 promoter.

Figures 2 to 4, 6 and 10 show the imaging results obtained in adult transgenic animals, with related diagrams as described below.

Figures 5, 7, 8 and 9 show further diagrams as described below.

### EXPERIMENTAL PART

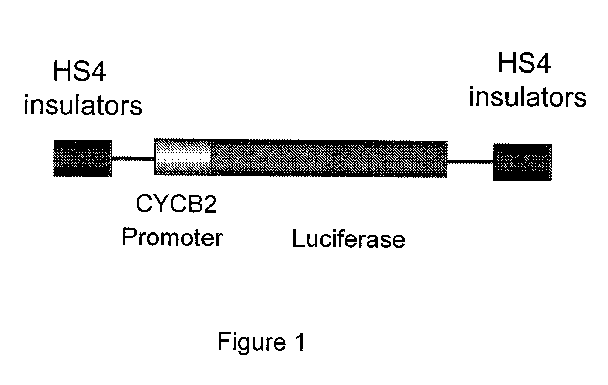

Figure 1 shows a diagram of the construct consisting of the nucleotide sequence used for transgenesis in order to obtain an animal line of the invention, which is preferably a mouse line, consisting of a reporter system composed of a gene coding the enzyme firefly luciferase (Photinus pyralis), corresponding to SEQ ID NO:2, under transcriptional control of a 312 bp fragment derived from the promoter of the gene encoding cyclin B2 (SEQ ID NO: 1), identified by the acronym CYCB2. The transgene was constructed, linearized, separated from plasmid sequences and microinjected into fertilized eggs of C57BI/6xDBA/2 using standard protocols. A total of 160 microinjected zygotes were implanted in the oviducts of 6 recipient mice that gave birth to 43 pups. To identify founder animals, genomic DNA was extracted from tail biopsies of the 43 animals and genotypic analysis was performed by PCR with oligonucleotide primers specific for the sequence of the transgene. Tail fragments were lysed overnight at 50 °C in buffer containing proteinase K and DNA was purified by the standard procedure based on extraction with phenol, chloroform and alcohol (Sambrook et al, 1989). The PCR reaction was performed using oligonucleotides with sequence:
forward oligo 5'TGTAGACAAGGAAACAACAAAGCCTGGTGGCC (SEQ ID NO: 5)
reverse oligo:5'GGCGTCTTCCATTTTACCAACAGTACCGG (SEQ ID NO: 6)

Of 43 individuals analyzed, two were identified as carriers of the transgene, these founders were crossed with wild-type mice and the heterozygous offspring was genotyped by PCR as described above.

### Characterization and validation of the model

The tissues of the transgenic mouse according to the invention express luciferase only in actively proliferating cells.

The luciferase activity was monitored in animal and embryo tissues by bioluminescence imaging or by determination of enzymatic activity in protein extracts (Bisemi A, Giannessi F, Sciarroni AF, Milazzo FM, Maggi A, Ciana P, In vivo imaging reveals selective peroxisome proliferator activated receptor modulator activity of the synthetic ligand 3-(1-(4-chlorobenzyl)-3-t-butylthio-5-isopropylindol-2-yl)-2,2-dimethylpropanoic acid (MK-886). Mol Pharmacol. 2008 May; 73(5):1434-43).

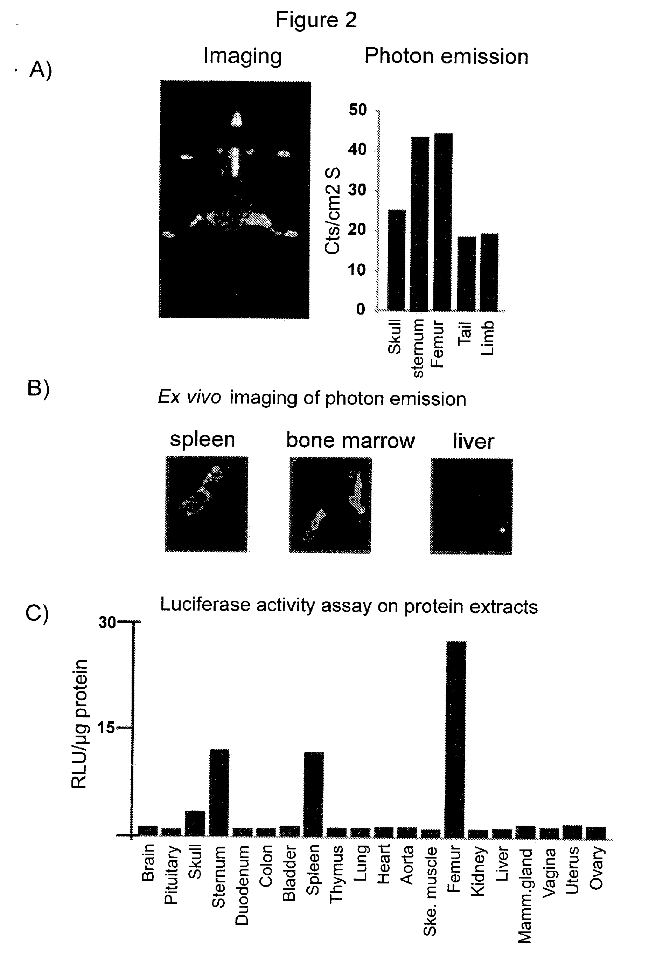

Bioluminescence imaging experiments on alive animals were conducted in heterozygous males 2-4 months of age. Mice were anesthesized by subcutaneous injection ketamine/xylazine and were administered one intraperitoneal dose of luciferin substrate (50 ug/kg). Fifteen minutes after luciferin injection (Bisemi et al., 2008), the luminescence emitted by the animals was measured by a CCD camera (Berthold Technologies, Wilbad) and superimposed with images in reflected light to locate the emission sources. As shown in Figure 2A, where the result of imaging is shown on the left and the photon emission graph is on the right, in the adult the distribution of photon emission is selective in some areas of the body. In particular, the emission is detected in the area corresponding to the chest (stemum), posterior limbs (femur) and areas of bone remodeling in the feet The identity of the source of light emission was confirmed by removing the organs beneath the signal and *ex vivo* measurement of light emission (Figure 2B). Finally, imaging data were confirmed by the analysis of enzyme activity in protein extracts, according to the graph shown in Figure 2C.

For this purpose, organs were homogenized in lysis buffer and lysates were subjected to three cycles of freezing and thawing. Proteins were separated from DNA and lysosomes by centrifugation (13000xg for 30 min). After determining the protein concentration of the extract, the biochemical assay of luciferase activity was carried out by measuring luminescence emission with a luminometer. The relative luminescence units (RLU) determined during a measurement of 10 seconds were then expressed on the Y axis as RLU values per microgram protein. The results according to the graph in Figure 2C indicate that, in the adult animal, light emission is restricted to organs characterized by basal homeostatic proliferation such as spleen, bone marrow, femur and sternum, or sites of active tissue remodeling in the mouse, such as joints of the feet, jaw, etc.

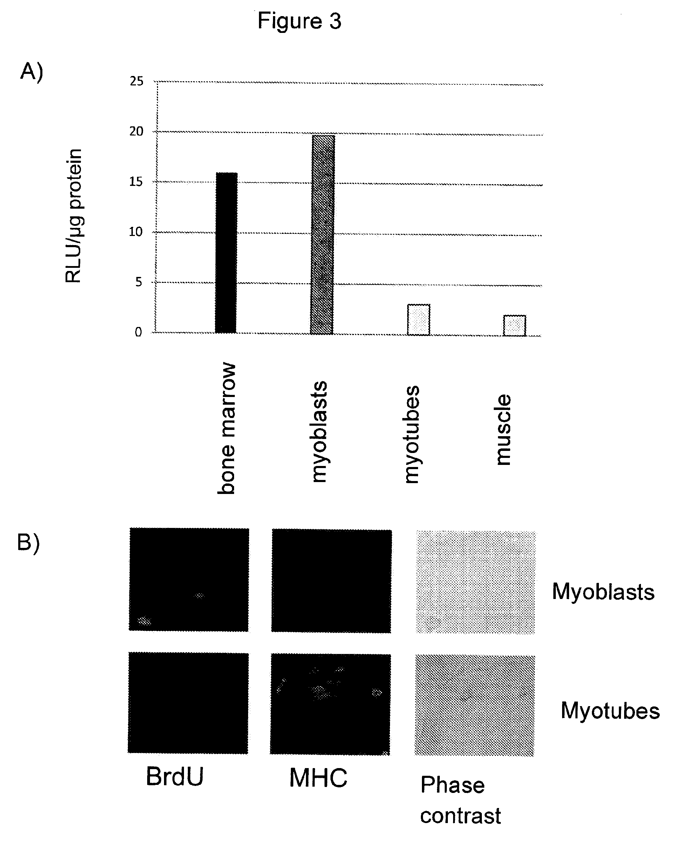

To assess the expression of luciferase in proliferating progenitor cells, bone marrow cells, that are highly enriched in hematopoietic precursors, were collected by flushing the femurs with culture medium and enzyme activity was analyzed in protein extracts (Ciana et al 2001), as shown in the graph in Figure 3A. From these data it can be concluded that, in the transgenic mouse according to the invention, luciferase is expressed in actively proliferating precursor cells.

Another actively proliferating histological type of precursor cell is represented by muscle satellite cells grown In the presence of growth factors. Muscle satellite cells were obtained by previously described protocols: Gurtner A, Fuschi P, Magi F, Colussi C, Gaetano C, Dobbelstein M, Sacchi A, Piaggio G. NF-Y dependent epigenetic modifications discriminate between proliferating and postmitotic tissue. PLoS One. 2008 Apr 23;3(4):e2047. Rando TA, Blau HM, Primary mouse myoblast purification, characterization, and transplantation for cell-mediated gene therapy. J Cell Biol. 1994 Jun;125(6):1275-87.

Cells were obtained from muscles of the front and hind limbs of newborn mice at 20 days of life. After removing bones, the muscle mass was subjected to enzymatic and mechanical dissociation. The resultant suspension was filtered and then centrifuged to pellet cells in suspension. The cell pellet was resuspended in culture medium enriched with growth factors (20% bovine serum, FGF, 3% embryo extract from fertilized eggs) and seeded in culture dishes to induce proliferation (myoblasts). The same cells were also seeded In collagen-containing plates and grown in culture medium devoid of growth factors (without serum) to induce muscle differentiation (myotubes). In addition, cytosine arabinoside, at a concentration of 50 µM, was added to the culture medium without serum to eliminate undifferentiated cells still proliferating. As demonstrated by the analysis of the enzymatic activity present in protein extracts, myoblasts express high levels of luciferase. In contrast, the expression of the enzyme in myotubes is reduced in the assay to undetectable levels similar to those found in differentiated muscle tissue (Figure 3A). Myoblasts used in this essay are highly proliferating, as demonstrated by their ability to incorporate bromodeoxyuridine (BrdU), a thymine analog that is incorporated in newly synthesized DNA, hence a marker to identify actively proliferating cells. In immunofluorescence assays, antibodies against BrdU were used to label cells that incorporated BrdU. In contrast myotubes do not incorporate BrdU, thus demonstrating that they are not proliferating. In addition, as expected, proliferating myoblasts do not express markers typical of differentiated muscle cells, such as MHC (Myosin Heavy Chain). The myotube differentiation index is high, as demonstrated by MHC expression shown in Figure 3B.

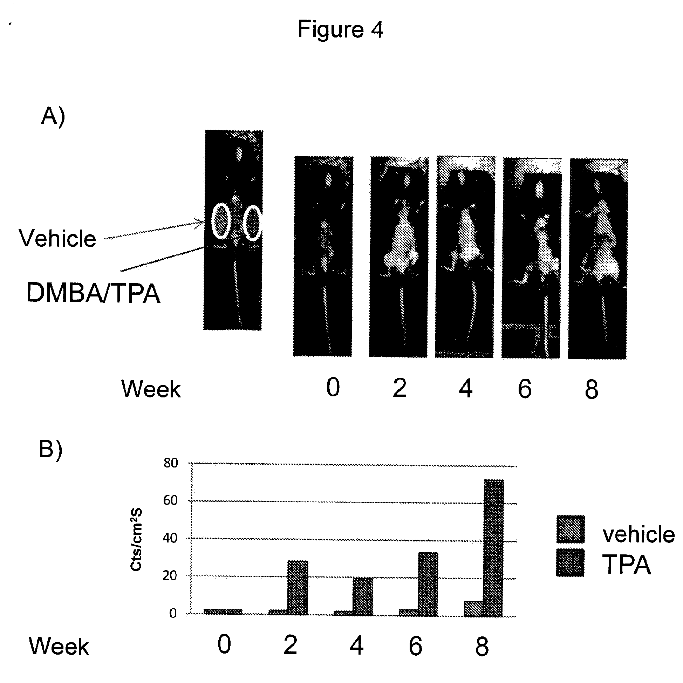

To further demonstrate that in the transgenic mouse according to the invention luciferase expression is correlated with cell proliferation, we applied a protocol that was used to induce skin hyperproliferation and thereby skin papillomas. The protocol involves cutaneous application of 0,24% 7,12-dimethylbenz(a)anthracene (DMBA) solution in acetone for a week, and of 5 nmoles of 12-O-tetradecanoylphorbol-13-acetate (TPA) weekly for a time sufficient to obtain palpable skin papillomas (about 3 months). Tumor promoters were applied to the ventral right region, while only the vehicle was applied to the left area: animals were subjected to the imaging procedure every two weeks up to 8 weeks, obtaining the results shown in Figure 4A. Increased light emission is observed in the area treated with DMBA and TPA, while the emission of photons remains roughly constant over time in the contralateral area treated with vehicle alone. The graph in Figure 4B shows how the intensity of the effect varies over time.

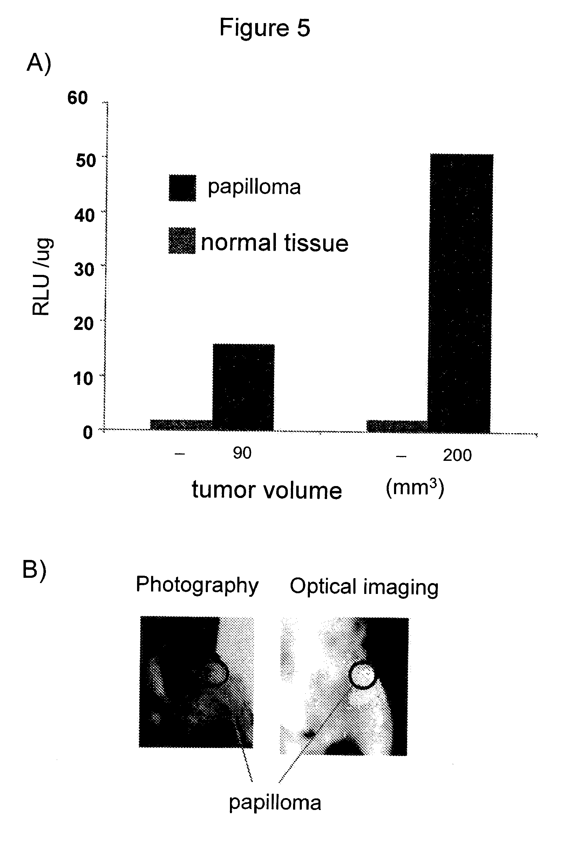

In agreement with these findings, the protein extracts from tumors that developed after treatment have a significantly higher luciferase activity compared to the normal tissue, as shown in Figure 5. In the graph in Figure 5A, it is interesting to note that the relative amount of luciferase is higher in the larger tumor compared to the smaller tumor; this difference apparently reveals that a larger amount of proliferating cells are present in the larger tumor. Figure 5B shows the presence of the papilloma, both by photography and optical imaging.

### Ubiquitous activity

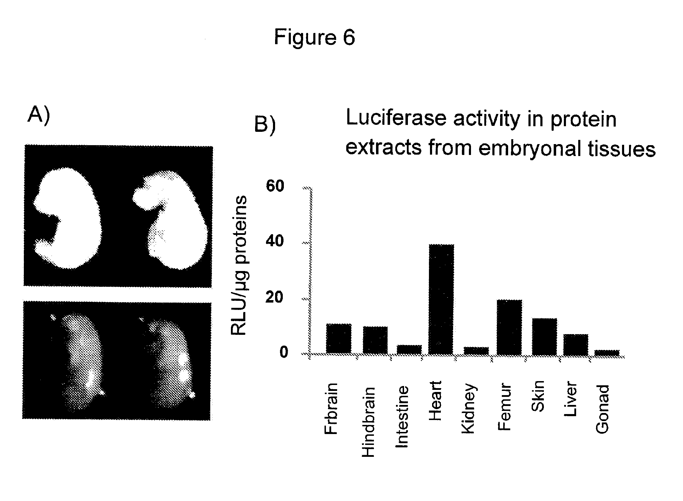

Finally, luciferase expression was analyzed in mouse embryos at the stage of 19 days post-conception, where cells of all tissues show a strong proliferative activity. Expression of the reporter gene was assessed by *ex vivo* imaging analysis performed on the whole embryo, shown in Figure 6A. For this purpose, wild type C57/BI6 pregnant females impregnated by transgenic males were treated with a single intraperitoneal injection of 150 ug/kg luciferin; 15 minutes later the animal was sacrificed and embryos were subjected to bioluminescence analysis. As shown in Figure 6B, all embryonic tissues analyzed, as specified therein, show a generalized light emission, a conclusion proven by an analysis of luciferase activity carried out on all tissues that could be isolated by micro-dissection.

Thus, the biosensor is active in all proliferating cells.

### Activity of compounds modulating proliferation

The reporter mouse generated according to the invention, as described above, is used as a tool to evaluate natural or synthetic molecules with pro- or anti-proliferative activity. Therefore the reporter mouse enables simultaneous quantification of the activity of a compound on cell proliferation in all tissues and in the physiological context. The method can be applied to:
1) development of compounds of pharmacological interest inhibiting cellular proliferation, such as antitumor compounds, anti-inflammatory compounds, etc.
2) development of compounds of pharmacological interest inducing cell proliferation, such as growth factors that selectively stimulate stem cell proliferation.
3) toxicological evaluation of compounds with potential tumorigenic activity, as well as assessment of compounds with chemopreventive anticancer activity.

### 1) Activity of compounds inhibiting proliferation

As an example of application for development of anti-proliferative drugs, heterozygous individuals of the transgenic mouse line according to the invention were treated as follows: a first group was treated with 150 ug/kg 5-fluorouracil (5FU), a well-known chemotherapeutic agent, while another group was treated with saline as control (vehicle) for 2 and 5 days.

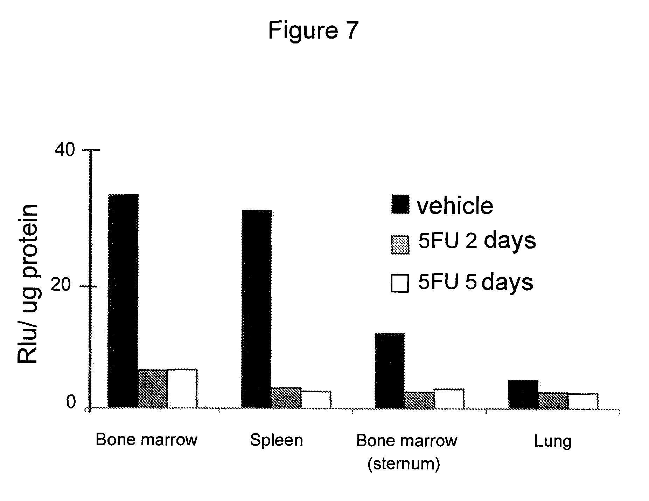

Mice were sacrificed and luciferase activity was evaluated in tissue protein extracts, as reported in the graph In Figure 7. Reduced luciferase activity, until levels that were undetectable by the method of analysis, were found in all the tissues of 5FU treated mice that were subjected to analysis.

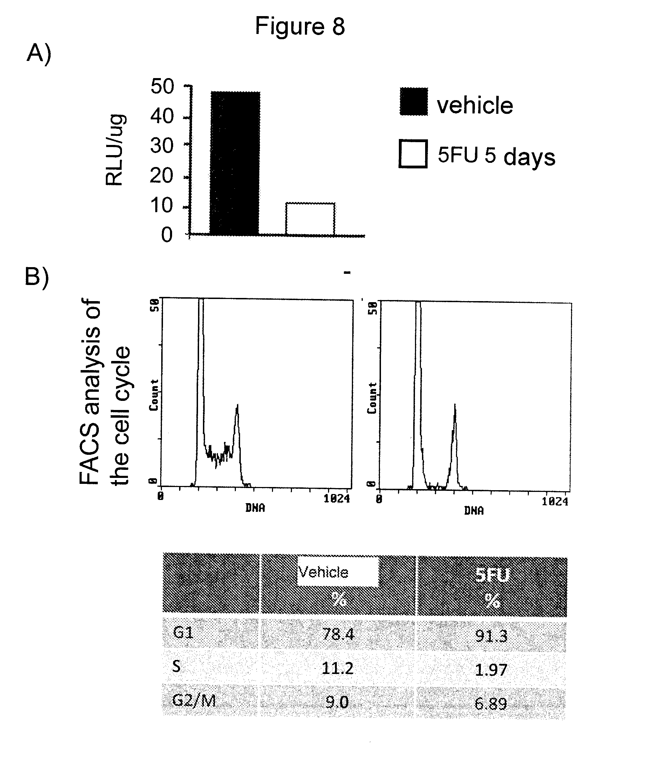

To demonstrate that the reduction of luciferase was related to reduced proliferation, bone marrow cells from mice treated with vehicle or 5FU were analyzed by detecting the cell cycle phase, using a methodology based on flow cytometry (FACS) and cell staining with propidium iodide. Cells were fixed with methanol acetone 4:1, stained with 0.1 mg/ml propidium iodide, a DNA fluorescent marker, and then subjected to FACS analysis. The graph in Figure 8B shows that 5FU treatment induces a cell cycle block Indicated by a decrease to 1.97% of S phase cells compared to a corresponding value of 11.2% detected in the sample treated with vehicle alone. These data demonstrate that it is possible to quantify the activity of compounds that reduce cell proliferation in tissues of the transgenic mouse according to the invention.

Data in the graph representing RLUs as function of microgram protein, in Figure 8A, demonstrate that a 5 day 5FU treatment inhibited proliferation and correspondingly reduced luciferase expression also in bone marrow cells.

### 2) Activity of compounds inducing proliferation

As discussed above, the data shown in Figure 3 on luciferase expression in proliferating progenitor cells and not in differentiated cells are an example of the application of the reporter mouse according to the invention to evaluate the action of growth factors on normal progenitor cells. As shown by the experiment, precursor stem cells placed in medium enriched with growth factors maintain stem cell properties, proliferate and express luciferase, whereas, if these precursors are placed in differentiation medium, cells differentiate, undergo cell cycle arrest and correspondingly lose the expression of the reporter.

### 3) Toxicological evaluation of compounds with potential tumorigenic activity

A further application of the transgenic animal according to the invention Is in the toxicological field. In particular, the ability to detect in tissues of the transgenic animal according to the invention a potential proliferative activity of a given compound, alone or in combination with other compounds, makes possible to delineate a map of the potential pro-carcinogenic activity.

To evaluate this application, the skin of a transgenic mouse according to the invention was treated with DMBA, a carcinogen, and the stimulation of light emission was measured for the carcinogen-treated side compared to the side treated with vehicle alone.

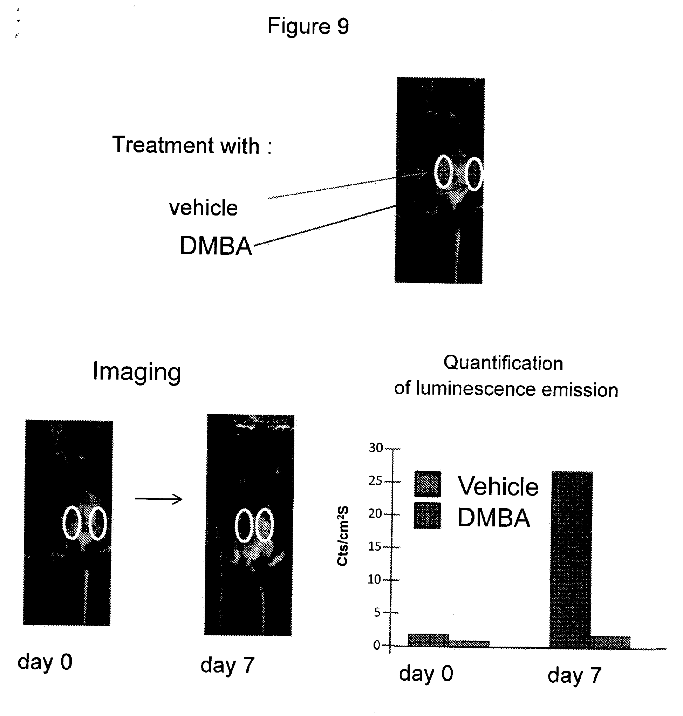

In particular, a 0.24% DMBA solution in acetone was applied to the skin of transgenic individuals according to the invention. One week later (day 7 relative to day 0) the amount of luciferase produced was detected by measuring the light emission produced by the skin of the animals. Figure 9 shows the corresponding imaging data.

The graph in Figure 9 shows a 12.5 fold induction of luciferase activity in the region of absorption of the compound compared to the bilateral region treated with vehicle alone, thus showing that the biosensor can detect the activity of carcinogens at an early stage, long before the development of a full-blown cancer. From the overall description given above, it is concluded that the transgenic animal according to the present invention provides an effective method to delineate the full profile of pharmacological activity of modulators of cell proliferation, through a simple and direct measurement of the proliferative state of animal tissues, both under physiological conditions and following pharmacological treatment.

## Claims

1. Transgenic, non-human mammalian animal whose genome incorporates, as a biosensor of the activity of cellular proliferation modulators, a DNA sequence comprising the luciferase reporter transgene ligated downstream of a sequence of the CYCB2 gene promoter, said promoter/reporter sequence being flanked by HS4 insulator sequences at each of its 3' and 5' sides.

2. Animal according to Claim 1, **characterized in that** it is a mouse.

3. Use of the transgenic non-human mammal animal according to Claim 1, for studying and developing compounds provided with an inhibitory activity on cellular proliferation and which are of potential pharmaceutical interest.

4. Use according to Claim 3, for studying and developing compounds provided with anti-tumor activity,

5. Use according to Claim 3, for studying and developing compounds provided with anti-inflammatory activity.

6. Use of the transgenic non-human mammal animal according to Claim 1, for studying and developing compounds provided with an inducing activity on cellular proliferation and which are of potential pharmaceutical interest.

7. Use according to Claim 6 for studying and developing compounds which are of potential pharmaceutical interest as growth factors stimulating selective proliferation of stem cells.

8. Use of the transgenic non-human mammal animal according to Claim 1, for studying and for toxicologically evaluating compounds provided with tumorigenic activity.

9. Use of the transgenic non-human mammal animal according to Claim 1, for studying and evaluating compounds provided with chemo-preventive activity against tumor insurgence.

10. Method for evaluating the activity of a cellular proliferation modulator according to one or more of the previous claims, **characterized in that** it comprises the steps of: constructing a transgenic non-human mammal animal line by incorporating in its genome a DNA sequence comprising the luciferase reporter transgene ligated downstream of a sequence of the CYCB2 gene promoter, said promoter/reporter sequence being flanked by HS4 insulator sequences at each of its 3' and 5' sides; treating individuals of said transgenic animal line, or tissues or cells derived therefrom, with said cellular proliferation modulator; comparing the luminescence emission in individuals treated with said cellular proliferation modulator and in other non-treated individuals of the same transgenic animal line.

11. Method according to Claim 10 for evaluating the inhibitory activity on cellular proliferation, in particular on anti-tumor activity or anti-inflammatory activity, **characterized in that** it comprises the steps of: treating living individuals of said transgenic animal line, or tissues or cells derived therefrom, with said cellular proliferation modulatory agent; evaluating the amount of luciferase produced by the animal by *in vivo* imaging assays, or produced by its tissues and by its cells by *ex vivo* enzymatic activity assays; comparing the luminescence emission in individuals treated with said cellular proliferation modulatory agent and in other non-treated individuals of the same transgenic animal line.

12. Method according to Claim 10 for evaluating the inducing activity on cellular proliferation, in particular the activity of compounds stimulating selective proliferation of stem cells, **characterized in that** it comprises the steps of: treating living individuals of said transgenic animal line, or tissues or cells derived therefrom, with said cellular proliferation modulatory agent; evaluating the amount of luciferase produced by the animal by *in vivo* imaging assays, or produced by its tissues and by its cells by *ex vivo* enzymatic activity assays; comparing the luminescence emission in the individuals treated with said cellular proliferation modulator and in other non-treated individuals of the same transgenic animal line.

13. Method according to Claim 10 for evaluating the tumorigenic activity **characterized in that** it comprises the steps of: treating living individuals of said transgenic animal line, or tissues or cells derived therefrom, with said cellular proliferation modulatory agent; evaluating the amount of luciferase produced by the animal by *in vivo* imaging assays, or produced by its tissues and by its cells by *ex vivo* enzymatic activity assays; comparing the luminescence emission in the individuals treated with said cellular proliferation modulator and in other non-treated individuals of the same transgenic animal line.

14. Method according to Claim 10, for evaluating the chemo-preventive activity against tumor insurgence, **characterized in that** it comprises the steps of: treating living individuals of said transgenic animal line, or tissues or cells derived therefrom, with said cellular proliferation modulatory agent; evaluating the amount of luciferase produced by the animal by *in vivo* imaging assays, or produced by its tissues and by its cells by *ex vivo* enzymatic activity assays; comparing the luminescence emission In the individuals treated with said cellular proliferation modulator and in other non-treated individuals of the same transgenic animal line.
